# EUROPEAN PATENT APPLICATION

(11) **EP 4 497 836 A1**
(43) Date of publication of application: **29.01.2025**
(21) Application number: 23775342.1
(22) Date of filing: 23.03.2023
(51) Int. Cl.: C12Q 1/6886, C12N 5/09, C12N 15/85, A61P 35/00

(54) **METHOD FOR CONSTRUCTING GENE NETWORK THROUGH SINGLE-CELL TRANSCRIPTOME AND METHOD FOR DISCOVERING KEY GENE IN DIFFERENTIATION USING SAME**

(30) Priority: 23.03.2022 KR 20220036186
(71) Applicant: Korea Advanced Institute of Science and Technology, Daejeon 34141 (KR)
(72) Inventor: CHO, Kwang Hyun, Daejeon 34141 (KR); GONG, Jeong Ryeol, Daejeon 34141 (KR); LEE, Chun Kyung, Daejeon 34141 (KR); KIM, Hoon Min, Daejeon 34141 (KR); KIM, Ju Hee, Daejeon 34141 (KR)
(74) Representative: Potter Clarkson
(86) International application number: PCT/KR2023/003908
(87) International publication number: WO 2023/182847

(57) **Abstract**

The present invention relates to a method for constructing a gene network through a single-cell transcriptome and a method for discovering key genes in differentiation using same, and a composition for the prevention, alleviation, or treatment of colon cancer using the target discovered through the method. The method for constructing a gene network of key genes according to the present invention employs single-cell transcriptome data and thus can be applied to all single-cell transcriptome data. The combination of MYB/HDAC2/FOXA2 discovered upon application to colon cells can serve as a cancer treatment target that promotes the differentiation of colon cancer cells to revert same into differentiated normal cells.

## Description

### [Technical Field]

The present invention relates to a method for constructing a gene network through a single-cell transcriptome, a method for discovering key genes in differentiation using the same, and a composition for the prevention, alleviation, or treatment of colon cancer using a target discovered through the same.

### [Background Art]

Targeted drugs aim to treat cancer by directly inhibiting oncogenes or oncogenic pathways, but unexpected drug resistance often appears. Due to the complex dynamics of a cancer signaling network, which consists of complex feedback regulation, cancer cells may adapt to drug treatment by influencing the drug therapy to cause a fundamental limitation of targeted therapies.

Cancer cells have higher stemness than normal cells, which has become an important cause of cancer cell metastasis and drug resistance. There have been many attempts to control the stemness, but there is yet no system biology approach.

As single-cell transcriptome technology advances, various analysis methods have been developed, and there is research on the technology of constructing a gene network with logic that follows a pseudo time of single-cell transcriptome data. However, since the single-cell transcriptome data has large noise and it is difficult to infer logic through hidden dynamics in single-cell data, the development of technology of constructing a gene network with logic is still lacking.

### [Disclosure]

### [Technical Problem]

Under the above-described technical background, the present inventors constructed a gene network with unsupervised logic from single-cell transcriptome data. In addition, the present inventors invented a pipeline for controlling the gene network, and proposed a cancer treatment method by applying a differentiation synergy target of colon cells derived therefrom to colon cancer cells to revert the colon cancer cells into differentiated normal cells.

An object of the present invention is to provide a method for constructing a simulation network that simulates a differentiation process, and to provide a method for discovering key genes in differentiation based on the same.

Another object of the present invention is to provide a pharmaceutical composition for the prevention or treatment of colon cancer.

Yet another object of the present invention is to provide an anticancer adjuvant.

Yet another object of the present invention is to provide a food composition for the prevention or alleviation of colon cancer.

Yet another object of the present invention is to provide a method for inducing conversion of colon cancer cells into differentiated normal cells or normal-like cells.

Yet another object of the present invention is to provide a screening method of agents for treating colon cancer.

Yet another object of the present invention is to provide a method for treating colon cancer.

### [Technical Solution]

One aspect of the present invention provides a method for constructing a network of key genes including: a) sorting single cells according to a pseudo time series in which cells with high stemness differentiate into differentiated cells;
b) separating each single cell sorted in step a) into a turn on Pseudo-state where each gene is expressed and a turn off Pseudo-state where each gene is not expressed, according to characteristics of immature RNA (nascent RNA) in the nucleus and mature RNA (exonic RNA) in the cytoplasm using single-cell transcriptome data;
c) deriving gene pairs that affect mutual expression by dividing the single cells sorted according to the pseudo time in step a) into sections so that X cells are included per group over time and performing conditional mutual information (CMI) analysis between genes over time using a moving windows analysis method, wherein X = 100 to 500;
d) constructing candidate regulator data of target genes with mutual feedback by excluding gene pairs that are not direct gene regulatory pairs from among the gene pairs derived through step c) using a CisTarget DB;
e) generating a simulatable control relationship logic by applying a truth table prepared by discretizing the candidate regulator data of the target genes in step d) to all gene pairs in each pseudo-state; and
f) selecting a final target that achieves global stabilization by using a BNsimpleReduction algorithm in the generated regulation relationship logic of step e).

The mature RNA (exonic RNA) of step b) may be considered as the amount of transcription factor, which is a regulator, and the immature RNA (nascent RNA) may be considered as a gene regulated by the regulator.

The CMI analysis of step c) may be performed by dividing sections to include 400 cells and repeating moving window analysis.

When the binarization of step d) corresponds to a section that forms a curve where the number of unspliced reads/spliced reads increases with latent time, the section may be determined as turned on, State = 1, where the gene is expressed and the remaining sections may be determined as turned off, State = 0, where the gene is not expressed.

In the truth table of step e), the mature RNA (exonic RNA) may be input and the immature RNA (nascent RNA) may be output.

The final target for achieving the global stabilization of step f) may be a combination with a similarity score of 90% to 100% by comparing the similarity scores between the stable state and the desired state of the network and a small number of regulators.

Further, another aspect of the present invention provides a pharmaceutical composition for the prevention or treatment of colon cancer, including a MYB inhibitor, a histone deacetylase 2 (HDAC2) inhibitor, and a forkhead box protein A2 (FOXA2) inhibitor.

The MYB, HDAC2 and FOXA2 may be combinations of key regulators in the cancer cell differentiation process.

The inhibitor may be at least one selected from the group consisting of antisense oligonucleotide, small interference RNA (siRNA), short hairpin RNA (shRNA), microRNA (miRNA), and ribozyme, that bind complementarily to mRNA of a MYB, HDAC2 or FOXA2 gene.

The inhibitor may be at least one selected from the group consisting of a compound, a peptide, a peptide mimetic, a substrate analog, an aptamer, and an antibody, that specifically bind to a MYB, HDAC2 or FOXA2 protein.

The composition may induce reversion of cancer cells into differentiated normal cells or normal-like cells.

Yet another aspect of the present invention provides an anticancer adjuvant including a MYB inhibitor, a histone deacetylase 2 (HDAC2) inhibitor, and a forkhead box protein A2 (FOXA2) inhibitor.

Yet another aspect of the present invention provides a food composition for the prevention or alleviation of colon cancer, including a MYB inhibitor, a histone deacetylase 2 (HDAC2) inhibitor, and a forkhead box protein A2 (FOXA2) inhibitor.

Yet another aspect of the present invention provides a method for inducing conversion of colon cancer cells into differentiated normal cells or normal-like cells, including treating colon cancer cells with a MYB inhibitor, a histone deacetylase 2 (HDAC2) inhibitor, and a forkhead box protein A2 (FOXA2) inhibitor in vitro.

Yet another aspect of the present invention provides a screening method of a colon cancer therapeutic agent, including (a) treating colon cancer cells with a candidate substance; (b) measuring the expression levels of MYB, HDAC2 and FOXA2 in the colon cancer cells treated with the candidate substance; and (c) determining the candidate substance as an agent for treating colon cancer if the expression levels of MYB, HDAC2 and FOXA2 are lower than that of a control group untreated with the candidate substance.

Yet another aspect of the present invention provides a method for treating colon cancer, including administering to a subject a MYB inhibitor, a histone deacetylase 2 (HDAC2) inhibitor, and a forkhead box protein A2 (FOXA2) inhibitor.

### [Advantageous Effects]

According to the present invention, the method for constructing a gene network of key genes employs single-cell transcriptome data and thus can be applied to all single-cell transcriptome data. The combination of MYB/HDAC2/FOXA2 discovered upon application to colon cells can serve as a cancer treatment target that promotes the differentiation of colon cancer cells to revert same into differentiated normal cells.

### [Description of Drawings]

FIG. 1 is a diagram illustrating a process of defining a single cell in a process of changing from a high stemness state to a differentiation state as a pseudo-time state and defining a single cell at a pseudo time in a physically distinct pseudo-state.
   The pseudo-state is divided into reads containing unspliced reads found in the nucleus and reads containing spliced reads found in the cytoplasmic area. The unspliced read may mean a T = 0 state, and spliced read may mean a T = 1 state.
FIG. 2 is a diagram illustrating a process of integrating a subnetwork of moving windows constructed with a mutual feedback relation by performing conditional mutual information (CMI) analysis in moving windows constructed by dividing a single-cell state represented by a pseudo time into sections with a predefined number of cells and excluding gene pairs indirectly affecting expression using a CisTarget DB.
FIG. 3 illustrates a process of placing a gene activity level in two states, i.e., on (State = 1) and off (State = 0), locating two types of binary codes in different groups, and generating logic for the groups.
FIG. 4 illustrates a model of transcriptional dynamics for inference of Boolean logic models and a logic network model based on an input-output relationship using a Quine-McCluskey algorithm.
FIG. 5 is a diagram illustrating results of applying a constructed network model to a colon cells source, and generating a TF gene network of 17 genes that are strongly interconnected through strongly connected components (SCC).
FIG. 6 is a diagram illustrating a simulation of a logic network consisting of 13 genes derived by applying a BNsimpleReduction algorithm developed by the present inventors to the TF gene network consisting of 17 genes. In the logic network consisting of 13 genes, the on-off interactions of the respective genes may be visualized as shown on the right.

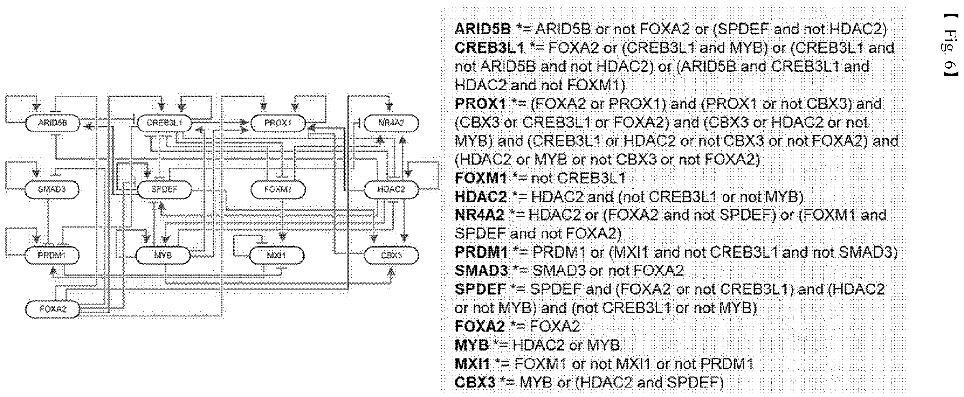

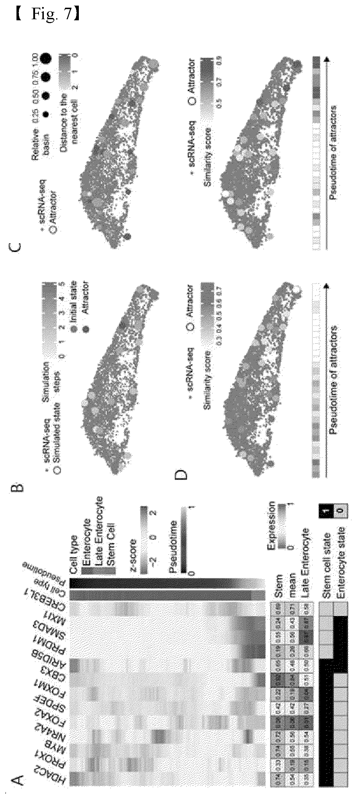

FIG. 7 is a diagram illustrating results of verification by comparing and analyzing the logic network of FIG. 6 with the dynamics of the used single-cell data. As a result of randomly simulating the logic network of FIG. 6 on the used single-cell data, it was verified that the constructed gene network model dynamics closely mimicked a cell differentiation process. A: Determining a binary state of each cell type, B: The simulation process is consistent with a pseudo-time direction, C: The state of a processing logic network is similar to a real cell state, D: The distance measurement results of the enterocyte vector and the simulation result show that the results for the directions of stem cells and enterocytes are consistent.

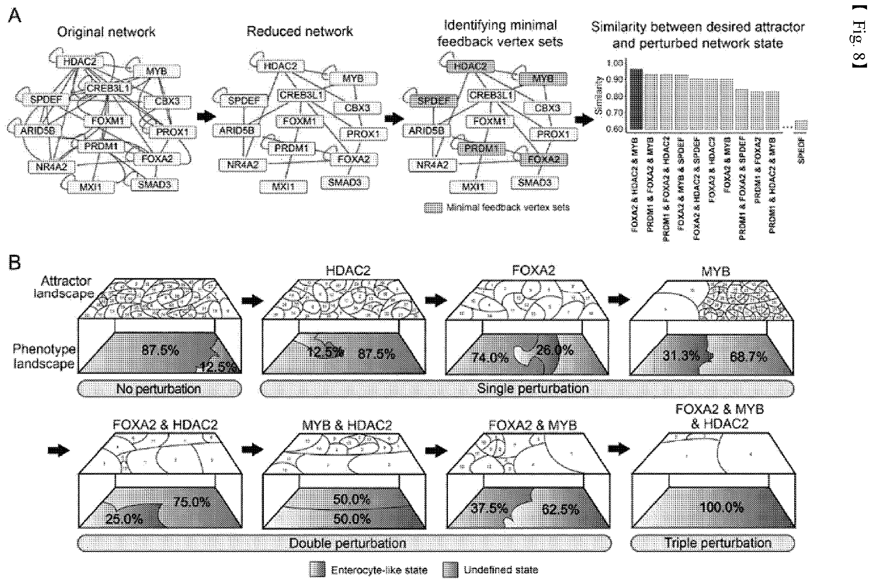

FIG. 8 is a diagram illustrating results of confirming that when three targets MYB, HDAC2, and FOXA2 are turned off simultaneously, the highest similarity to a desired state (enterocyte point attractor) as a result of simulating all possible combinations of five targets discovered by applying an algorithm discovering control targets to a gene network derived as a reduced network using BNSimpleReduction (A), and confirming that only the simultaneous application of the three targets may converge the gene network point attractors to a desired state as a result of confirming a phenotype landscape of the gene network.
FIG. 9 is a diagram illustrating results of significantly reducing the cell proliferation when MYB, HDAC2 and FOXA2 are simultaneously inhibited (siMYB/HDAC2/FOXA2) all in (a) HCT 116 cell line, (b) HT 29 cell line, and (c) CACO 2 cell line.
FIG. 10 is a diagram illustrating crystal violet staining results of significantly reducing the cell proliferation when MYB, HDAC2 and FOXA2 are simultaneously inhibited (siMYB/HDAC2/FOXA2) all in (a) HCT 116 cell line, (b) HT 29 cell line, and (c) CACO 2 cell line.
FIG. 11 is a diagram illustrating results of confirming that there is a synergistic effect in which the expression of enterocyte differentiation markers is increased when MYB, HDAC2 and FOXA2 are simultaneously inhibited (siMYB/HDAC2/FOXA2) all in (a) HCT 116 cell line, (b) HT 29 cell line, and (c) CACO 2 cell line.
FIG. 12 is a diagram illustrating a synergistic effect in which tumor formation and growth are significantly inhibited compared to a control group in both (a) mice transplanted with a HCT 116 cell line treated with shMYB/HDAC2/FOXA2 and (b) mice transplanted with a HT 29 cell line treated with shMYB/HDAC2/FOXA2.

### [Best Mode of the Invention]

Unless otherwise contrarily defined, all terms used herein including technological or scientific terms have the same meanings as those generally understood by a person with ordinary skill in the art.

Terms which are defined in a generally used dictionary should be interpreted to have the same meaning as the meaning in the context of the related art, and are not interpreted as an ideal meaning or excessively formal meanings unless otherwise clearly defined in the present application. Further, in describing the present invention, a detailed explanation of known related technologies may be omitted to avoid unnecessarily obscuring the subject matter of the present invention.

The present inventors invented a pipeline for constructing a gene network with unsupervised logic in single-cell transcriptome data, and proposed a cancer treatment method through reverting colon cancer cells into differentiated normal cells by applying a differentiated synergy target of cancer cells derived therefrom to colon cancer.

The present invention relates to a method for constructing a network of key genes including: a) sorting single cells according to a pseudo time in which cells with high stemness differentiate into differentiated cells;
b) separating each single cell sorted in step a) into a turn on Pseudo-state where each gene is expressed and a turn off Pseudo-state where each gene is not expressed, according to characteristics of immature RNA (nascent RNA) in the nucleus and mature RNA (exonic RNA) in the cytoplasm using single-cell transcriptome data;
c) deriving gene pairs that affect mutual expression by dividing the single cells sorted according to the pseudo time in step a) into sections so that X cells are included per group over time and performing conditional mutual information (CMI) analysis between genes over time using a moving windows analysis method, wherein X = 100 to 500;
d) constructing candidate regulator data of target genes with mutual feedback by excluding gene pairs that are not direct gene regulatory pairs from among the gene pairs derived through step c) using a CisTarget DB;
e) generating a simulatable control relationship logic by applying a truth table prepared by discretizing the candidate regulator data of the target genes in step d) to all gene pairs in each pseudo-state; and
f) selecting a final target that achieves global stabilization by using a BNsimpleReduction algorithm in the generated regulation relationship logic of step e).

Cancer cells have higher stemness than normal cells, which has become an important cause of cancer cell metastasis and drug resistance. There was derived a method capable of inferring the interaction logic of genes involved in a process of inducing the reversion of these cancer cells into normal cells or normal-like cells.

A differentiation process of cancer cells is a process of changing from a high stemness state to a differentiation state, and gene expression changes during this process. The differentiation state may mean that the cells become enterocytes.

As used in the present invention, the term 'pseudo time' refers to a latent (unobserved) dimension that measures the progress of cells. Pseudo time inference may aim to infer the order of cells along a lineage based on gene expression profiles of cells measured by scRNA-seq.

In a conventional process of constructing a gene regulatory network using a single-cell transcriptome, the front and back of single cells lined up according to the pseudo time inference are compared to identify a changing process in each single cell, and in this process, pairs of multiple cells were observed to construct a gene regulatory network based on the observation. However, up until now, the method has been inaccurate in finding changes in genes in each pair because the size of the changing time for each state is different, even though it identifies differences in genes over time that change in the process. A method of inferring a mechanism of cell movement by calculating change values during the cell change process is enabled under the assumption that the intervals of the cell change process are the same. However, when cells move dynamically, the intervals are increased and cells that are not captured occur, and thus there is a problem in that the genetic dynamics during the cell change process are not reflected.

Accordingly, to compensate for these shortcomings, the present inventors developed a method for inferring logic that expresses the dynamics of genes based on changes of a process of dividing single cells sorted according to pseudo time into two nucleus and cytoplasm, like two cells physically separating a state in which genes are changed by gene regulators within cells rather than between cells of pairs of each change by using single-cell transcriptome data and changing from RNA that starts to be produced inside the nucleus of each cell, that is, nascent RNA (immature RNA), to mature RNA based thereon. This method has never been developed before, and through this, a method for constructing a more accurate gene regulatory network from single-cell transcriptome data.

The single-cell transcriptome data may be time-series transcriptome data obtained from RNAseq, but are not limited thereto.

As used in the present invention, the term 'pseudo-state' may mean a state of a cell according to pseudo time. The pseudo-state may be characterized by the nucleus and the cytoplasm, which are physically separated during the cell change process, that is, nascent RNA that begins to be produced inside the nucleus and mature mRNA that is translated into actual proteins in the cytoplasm. In the present invention, each pseudo cell is set based on the characteristics of two mRNAs, which have different characteristics, which is named Pseudo-state.

In the pseudo-state, the mature RNA may be considered as the amount of a regulator, i.e. a transcription factor (TF). Based on this, the amount of mRNA that binds to TF to induce expression may be estimated, and it is assumed that the nascent RNA (immature RNA) that begins to be produced inside the nucleus of each cell is a gene regulated by the previously inferred transcription factor (TF).

Based on this, it is possible to infer logic that expresses the dynamics of genes based on a process in which nascent RNA, i.e. immature RNA, which begins to be produced inside the nucleus of each cell, is transcribed by the transcription factor, construct a gene network by integrating the logic at a system level, and find candidate regulators of target genes by analyzing the network.

The process of inferring gene pairs that affect the mutual expression between genes of each pseudo-state may use conditional mutual information (CMI) analysis, but is not limited thereto.

The method of calculating the CMI may more accurately calculate the effect of the regulatory relationship between a source gene and an actual target gene on a change in mature RNA expression level by considering the amount of change in target gene expression to be naturally changed after a unit of time, than methods for constructing other networks used commonly and infer a directional regulatory causal relationship.

The CMI analysis may be performed through moving windows that divide sections to include 100 to 500 cells, preferably 400 cells, and integrate each section into a constructed network.

Each section shares some cells with the previous and subsequent sections. In this process, n networks constructed for each section are merged and used as a single network. The network constructed in this way includes not only a regulatory relationship that appears consistently throughout the entire network, but also a regulatory relationship that appears characteristically in each section. That is, when the gene determines the state of any cell, both the early and late regulatory relationships of the gene are important, and when CMI is analyzed entirely without dividing the sections, it is possible to solve a problem in which a feedback correlation of the gene regulatory relationship is not properly reflected.

The candidate regulator data of target genes with mutual feedback may be constructed by excluding gene pairs that are not direct gene regulatory pairs from among the gene pairs derived through the CMI analysis using a CisTarget DB.

The candidate regulators of the target genes are gene pairs having direct links that regulate mutual expression, and the data constructed in this way may isolate genes that have indirect links that are not filtered out and reconstruct gene pair data that have strong connectivity.

In an example of the present invention, a strongly connected component (SCC) was used, but is not limited thereto. Depending on the single-cell transcriptome data used, networks commonly used in the art may be appropriately selected.

In the next process, simulatable control relationship logic may be generated by applying a truth table prepared by discretizing the candidate regulator data of the target genes to all gene pairs in each pseudo-state.

In the truth table, the mature RNA (exonic RNA) may be input and the immature RNA (nascent RNA) may be output.

When preparing the truth table, rows with the same input but different outputs may occur frequently, but rows with a small number thereof may be considered noise, so that the truth table was completed by selecting a plurality of input-output rows. A Boolean function may be created by grouping Boolean variables in each row of the truth table with 'AND' and grouping each row with 'OR', but in this case, because the length becomes very long, a minimal Boolean expression is made through a QM algorithm, which is an algorithm for reducing the Boolean expression to ultimately make a final Boolean function.

When the binarization corresponds to a section that forms a curve where the number of unspliced reads/spliced reads increases with latent time, the section may be determined as turned on, State = 1, where the gene is expressed and the remaining sections may be determined as turned off, State = 0, where the gene is not expressed.

By performing binary conversion on each transcription factor (TF) involved in the moment when RNA is expressed or not expressed, a logic form may be constructed as 1 when the gene is expressed and 0 when the gene is not expressed depending on a pattern of TFs expressed as 1 or 0. Through this, when a certain type of TF is present, a pattern of whether a gene is expressed (1) or not expressed (0) is derived. The presence of gene expression may be identified according to a pattern of each transcription factor with 99% accuracy when performed on approximately 2,000 or more pairs.

When the process is repeated for all genes in each pseudo-state to be applied to all pairs and the desired state of the cell is fixed in this process and applied with a BNsimpleReduction algorithm, it is possible to generate regulatory relationship logic of genes closely involved in the desired state.

According to an example of the present invention, a regulatory relationship logic network model consisting of 13 genes was constructed as a result of applying colon cell samples to single cell transcriptome data according to a network constructing method. When the dynamics of the network model constructed in this way were compared with the dynamics of the single-cell data used, it was confirmed that the dynamics were well reflected in transient dynamics converging from a single initial state of the network model to a stable state (attractor).

These results mean that the constructed network model may simulate combinations of key gene regulators to be converted to a desired state.

Therefore, a network model constructed by a method for constructing a key gene simulation network according to the present invention may simulate whether a desired state is achieved when a certain combination of genes is fixed to 0 and 1 in silico performed through a computer.

In the next process, the final target that achieves global stabilization may be selected by using the BNsimpleReduction algorithm on the generated regulatory relationship logic.

As used in the present invention, the term 'global stabilization' means a network control strategy that converges the network to a specific stable state specified for all possible initial states.

The final target for achieving the global stabilization may be a combination with a similarity score of 90% to 100% by comparing the similarity scores between the stable state and the desired state of the network and a small number of regulators.

As used in the present invention, the term 'stable state of the network' means a stable state in which the network finally converges through state transition according to an update rule.

As used in the present invention, the term 'desired state' means a cell state that exists in a network and is desired by a researcher using the present patent. **In** the present invention, the desired state may mean a state in which the cells have differentiated into enterocytes.

According to an example of the present invention, as a result of each simulation by applying an algorithm discovering control targets in a gene network derived as a reduced network using BNSimpleReduction to apply all possible combinations of the five discovered targets, when calculating the similarity score between the stable state and the desired state (enterocyte point attractor) of the network, the similarity score of approximately 95% or more was achieved by turning off three optimal targets MYB, HDAC2, and FOXA2.

As a result of confirming a phenotype landscape of the network while increasing the number of optimal three-target combinations, as shown in FIG. 8B, it was confirmed that convergence to the desired state (enterocyte point attractor) did not occur with a single or two combinations, and only simultaneous application of three targets MYB, HDAC2, and FOXA2 converges undifferentiated network point attractors to the desired state (enterocyte point attractor).

Through the process, MYB/HDAC2/FOXA2 differentiation regulatory targets were discovered, and through cell experiments and animal experiments, it was verified that simultaneous inhibition of the MYB/HDAC2/FOXA2 differentiation regulatory targets had effects of inhibiting the proliferation of cancer cells, increasing enterocyte differentiation markers, and inhibiting tumor formation or growth.

Therefore, in another aspect, the present invention relates to a pharmaceutical composition for the prevention or treatment of colon cancer, including a MYB inhibitor, a histone deacetylase 2 (HDAC2) inhibitor, and a forkhead box protein A2 (FOXA2) inhibitor.

The MYB, HDAC2 and FOXA2 may be combinations of key regulators in the cancer cell differentiation process.

According to an example of the present invention, the effects of inhibiting the proliferation of colon cancer cell lines in which the expression of MYB, HDAC2 and FOXA2 is simultaneously inhibited and increasing the expression of enterocyte differentiation markers were confirmed, and an animal model transplanted with a colon cancer cell line in which the combinations of MYB, HDAC2, and FOXA2 were simultaneously inhibited had an effect in which tumors were not formed or tumor growth was inhibited. Therefore, the pharmaceutical composition including the MYB inhibitor, the HDAC2 inhibitor, and the FOXA2 inhibitor according to the present invention as active ingredients may be provided for the prevention or treatment of colon cancer.

The inhibitor of the present invention may use any agent or means known in the art, as long as the inhibitor may reduce the MYB, HDAC2 or FOXA2 expression level or activity in cancer cells, as the purpose of the present invention.

Preferably, the inhibitor may be at least one selected from the group consisting of antisense oligonucleotide, small interference RNA (siRNA), short hairpin RNA (shRNA), microRNA (miRNA), and ribozyme that bind complementarily to mRNA of a MYB, HDAC2 or FOXA2 gene, but is not limited thereto.

As used in the present invention, the "antisense nucleic acid" refers to DNA, RNA, or derivatives thereof containing a nucleic acid sequence complementary to a specific mRNA sequence, and serves to inhibit the translation to a protein of mRNA by binding to the complementary sequence in mRNA. The antisense sequence refers to a DNA or RNA sequence complementary to mRNA of the gene and capable of binding to the mRNA, and may inhibit translation of the mRNA, translocation into the cytoplasm, maturation, or all other essential activities for overall biological functions.

In addition, the antisense nucleic acid may be modified at one or more base, sugar or backbone positions to enhance the efficacy. The nucleic acid backbone may be modified with phosphorothioate, phosphotriester, methyl phosphonate, short-chain alkyl, cycloalkyl, short-chain heteroatomic, heterocyclic intersugar linkages, and the like. In addition, the antisense nucleic acid may include one or more substituted sugar moieties. The antisense nucleic acid may include modified bases. The modified bases include hypoxanthine, 6-methyladenine, 5-methylpyrimidine (particularly, 5-methylcytosine), 5-hydroxymethylcytosine (HMC), glycosyl HMC, gentobiosyl HMC, 2-aminoadenine, 2-thio uracil, 2-thiothymine, 5-bromouracil, 5-hydroxymethyluracil, 8-azaguanine, 7-deazaguanine, N6(6-aminohexyl)adenine, 2,6-diaminopurine, and the like. In addition, the antisense nucleic acid may chemically bind to one or more moieties or conjugates that improve the activity and cell adhesion of the antisense nucleic acid. The antisense nucleic acid includes fat-soluble moieties, such as cholesterol moiety, cholesteryl moiety, cholic acid, thioether, thiocholesterol, fatty chain, phospholipid, polyamine, polyethylene glycol chain, adamantane acetic acid, palmityl moiety, octadecylamine, hexylaminocarbonyl-oxycholesterol moiety, and the like, but is not limited thereto. The antisense oligonucleotide may be synthesized in vitro by a conventional method to be administered in vivo, or may be synthesized in vivo.

As used in the present invention, "siRNA" means a nucleic acid molecule capable of mediating RNA interference or gene silencing. Since the siRNA may inhibit the expression of a target gene, the siRNA is provided as an effective gene knockdown method or gene therapy method.

The siRNA molecule of the present invention may have a structure forming a double chain in which a sense strand (a sequence corresponding to an mRNA sequence of a MYB, HDAC2 or FOXA2 gene as a target gene) and an antisense strand (a complementary sequence to the mRNA sequence) are located opposite each other, and the siRNA molecule of the present invention may have a single-stranded structure with self-complementary sense and antisense strands. Furthermore, the siRNA is not limited to complete pairing of a double-stranded RNA portion paring RNAs, but may include a portion which is not paired by mismatch (corresponding bases are not complementary), bulge (there is no corresponding base on one chain), etc. In addition, when a siRNA end structure may suppress the expression of the target gene by an RNAi effect, both a blunt end and a cohesive end are possible, and the cohesive end structure is able to be both a 3'-end protruding structure and a 5'-end protruding structure.

In an example of the present invention, si-MYB represented by SEQ ID NOs: 1 and 2, si-HDAC2 represented by SEQ ID NOs: 3 and 4, and si-FOXA2 represented by SEQ ID NOs: 5 and 6 were used, and as long as the purpose of the present invention may be achieved, it is not limited thereto.

The "shRNA" of the present invention is called small hairpin RNA or short hairpin RNA, and is used for silencing the gene by RNA interference. Usually, the shRNA is introduced into a target cell using a vector. Such a shRNA hairpin structure is also cleaved by other intracellular substances to become siRNA.

In an example of the present invention, sh-MYB represented by SEQ ID NO: 35, sh-HDAC2 represented by SEQ ID NO: 36, and sh-FOXA2 represented by SEQ ID NO: 37 were used, and as long as the purpose of the present invention may be achieved, it is not limited thereto.

In addition, the present invention may include functional equivalents of the base sequence of the si-MYB, si-HDAC2, si-FOXA2, sh-MYB, sh-HDAC2 or sh-FOXA2. The "functional equivalent" refers to polynucleotide that exhibits substantially the same physiological activity as the polynucleotide represented by the base sequence represented by SEQ ID NO: 1 having sequence homology of at least 70% or more, preferably 80% or more, more preferably 90% or more, and much more preferably 95% or more with the base sequence of the si-MYB, si-HDAC2, si-FOXA2, sh-MYB, sh-HDAC2 or sh-FOXA2, as a result of deletion, substitution or insertion of bases. The "% of sequence homology" with the polynucleotide is determined by comparing two optimally arranged sequences with a comparison region, and a part of a polynucleotide sequence in the comparison region may include addition or deletion (i.e., gap) compared to a reference sequence (without including addition or deletion) for an optimal alignment of the two sequences.

As used while mentioning the change of cancer cells into differentiated normal cells or normal-like cells, the terms "reversion" and "conversion" mean that cancer cells are differentiated into normal cells or normal-like cells when the MYB, HDAC2 and FOXA2, which are target genes discovered in the present invention, are inhibited in cancer cells, and are used interchangeably in the present disclosure.

In the present invention, the "normal-like cell" refers to a cell having a transcriptome of a cancer cell-derived normal cell, and refers to a normal cell, preferably a cell with the same or similar activity as or to a normal cell.

According to an embodiment of the present invention, the reversion to differentiated normal cells or normal-like cells may be a change to a normal cell shape, restoration of normal cell functions, or induction of an epigenetic change to normal cells. In a preferred embodiment of the present invention, it was confirmed that when MYB, HDAC2 and FOXA2 were inhibited in colon cancer cells, the reversion to normal cells or normal-like cells was induced.

Accordingly, it is verified that the MYB, HDAC2 and FOXA2 inhibition of the present invention may reprogram or convert cancer cells into differentiated normal cells or normal-like cells.

Unlike conventional anticancer agents that kill cancer cells, when the MYB, HDAC2 and FOXA2 inhibitors of the present invention were treated on cancer cells, the MYB, HDAC2 and FOXA2 inhibitors may inhibit cancer cell characteristics of target cells, such as proliferation ability, growth ability, metastatic ability, invasion ability, or migration ability, and promote the natural shape and function of the target cells, such as a change to a normal cell shape, recovery of normal cell functions or induction of changes into normal cells, thereby exhibiting the same functions as normal cells by converting cancer cells into normal or normal-like cells again. Accordingly, unlike conventional anticancer agents that only induce cell death, it is possible to solve side effects caused by cell death.

As used in the present invention, the term "including as the active ingredient" means including the MYB, HDAC2 and FOXA2 inhibitors, which are the active ingredients of the present invention, in amounts sufficient to achieve a predetermined efficacy or activity. The MYB, HDAC2 and FOXA2 inhibitors may be administered in a pharmaceutically effective amount, and the effective amount level may be determined depending on the type, age, and sex of a subject, sensitivity to a drug, a treatment period, drugs used simultaneously, and other medical factors.

In the present invention, the pharmaceutical composition may be in the form of capsules, tablets, granules, injections, ointments, powders or beverages, and the pharmaceutical composition may target humans. The pharmaceutical composition is not limited thereto, but may be formulated and used in the form of oral formulations, such as powders, granules, capsules, tablets, aqueous suspensions, etc., external preparations, suppositories, and sterile injectable solutions according to a conventional method, respectively.

The pharmaceutical composition according to the present invention may include a pharmaceutically acceptable carrier. The pharmaceutically acceptable carrier may be used with a binder, a lubricant, a disintegrant, an excipient, a solubilizer, a dispersant, a stabilizer, a suspending agent, a pigment, a flavoring, and the like during oral administration, may be mixed and used with a buffering agent, a preservative, a painless agent, a solubilizer, an isotonic agent, a stabilizer, and the like in the case of injections, and may be used with a base, an excipient, a lubricant, and a preservative, and the like in the case of topical administration. The formulations of the pharmaceutical composition of the present invention may be prepared variously in combination with the pharmaceutically acceptable carrier described above.

For example, for oral administration, the pharmaceutical composition may be formulated in the form of tablets, troches, capsules, elixirs, suspensions, syrups, wafers, etc., and for injections, the pharmaceutical composition may be formulated into a single dose ampoule or a multiple dose form. In addition, the pharmaceutical composition may be formulated into solutions, suspensions, tablets, capsules, sustained release agents, and the like.

Meanwhile, examples of the carrier, the excipient, and the diluent suitable for the formulations may be used with lactose, dextrose, sucrose, sorbitol, mannitol, xylitol, erythritol, maltitol, starch, acacia gum, alginate, gelatin, calcium phosphate, calcium silicate, cellulose, methylcellulose, microcrystalline cellulose, polyvinylpyrrolidone, water, methylhydroxybenzoate, propylhydroxybenzoate, talc, magnesium stearate, mineral oils, or the like. **In** addition, the pharmaceutical composition may further include fillers, anti-coagulating agents, lubricants, wetting agents, flavorings, emulsifiers, preservatives, and the like.

The route of administration of the pharmaceutical composition according to the present invention is not limited thereto, but includes oral, intravenous, intramuscular, intraarterial, intramedullary, intrathecal, intracardiac, transdermal, subcutaneous, intraperitoneal, intranasal, intestinal, topical, sublingual or rectal administration. The "parenteral" includes subcutaneous, intradermal, intravenous, intramuscular, intraarticular, intrasynovial, intrasternal, intrathecal, intralesional and intracranial injection or infusion techniques. The pharmaceutical composition according to the present invention may also be administered in the form of a suppository for rectal administration.

The pharmaceutical composition according to the present invention may be variously changed according to various factors, including the activity of a specific active ingredient used, age, body weight, general health, sex, diet, administration time, administration route, excretion rate, drug combination, and the severity of a specific disease to be prevented or treated. The dose of the pharmaceutical composition varies depending on the condition, body weight, degree of a disease, drug form, and administration route and period of a patient, but may be appropriately selected by those skilled in the art. Preferably, the pharmaceutical composition may be administered with an amount capable of obtaining a maximum effect with a minimum amount without side effects in consideration of all the factors, and may be repeatedly administered in an effective amount of more preferably 1 to 10000 µg/weight kg/day, and much more preferably 10 to 1000 mg/weight kg/day several times a day. The dose does not limit the scope of the present invention in any aspect.

The pharmaceutical composition of the present invention may be used alone or in combination with surgery, radiation therapy, hormone therapy, chemotherapy, and methods of using biological response modifiers, for prevention or treatment of target indications.

In another aspect, the present invention relates to an anticancer adjuvant including a MYB inhibitor, an HDAC2 inhibitor and a FOXA2 inhibitor.

As used in the present invention, the anticancer adjuvant refers to an agent that may alleviate, improve, or increase the anticancer effect of the anticancer agent by administering in combination with the anticancer agent when administering the anticancer agent. In the present invention, the anticancer adjuvant may be used as an anticancer agent or an anticancer adjuvant depending on a treatment concentration, and may enhance the sensitivity of the anticancer agent.

The adjuvant of the present invention may be administered simultaneously, separately or sequentially with or from the anticancer drug. The order of administration of the anticancer adjuvant according to the present invention, that is, which of the anticancer agent and the anti-cancer adjuvant is administered at some point and simultaneously, individually or sequentially, may be determined by a doctor or expert. The order of administration may vary depending on many factors. The anticancer adjuvant may be administered in combination with known compounds that have the effect of preventing, alleviating, or treating cancer. In this regard, the anticancer adjuvant may be administered simultaneously or sequentially with known compounds.

The known compound may be at least one anticancer agent selected from the group consisting of oxaliplatin, 5-fluorouracil (5-FU), doxorubicin, irinotecan, carboplatin, paclitaxel, gemcitabine and bortezomib, but is not limited thereto.

In another aspect, the present invention provides a food composition for the prevention or alleviation of colon cancer including a MYB inhibitor, an HDAC2 inhibitor and a FOXA2 inhibitor.

The food composition according to the present invention includes all forms, such as functional food, nutritional supplements, health food, health supplements, and food additives. The type of food composition may be formulated in any one form selected from the group consisting of powders, tablets, capsules, pills, and liquids according to a conventional method known in the art, but is not limited thereto. The food composition may be formulated in various forms using methods known in the art.

For example, as the health food, the MYB, HDAC2, and FOXA2 inhibitors themselves of the present invention may be ingested by granulation, encapsulation and powder or prepared in the form of tea, juice, and drinks to be drunk. In addition, the MYB, HDAC2, and FOXA2 inhibitors of the present invention may be mixed with known substances or active ingredients known to have the prevention, alleviation, or treatment activity of cancer to be prepared in the form of a composition.

In addition, the functional food may be prepared by adding the MYB, HDAC2, and FOXA2 inhibitors of the present invention to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

In addition, the food composition of the present invention may include conventional food additives, and the suitability as the "food additive" is determined by the specifications and standards for the corresponding item in accordance with the general rules of the Food Additive Codex, general test methods, and the like approved by the Food and Drug Administration, unless otherwise specified. The items disclosed in the "Food Additives Codex" may include, for example, chemical composites such as ketones, glycine, calcium citrate, nicotinic acid, cinnamic acid, etc., natural additives such as persimmon color, licorice extract, crystal cellulose, Kaoliang color, guar gum, etc., and mixed formulations such as sodium L-glutamic acid formulations, alkali agents for noodles, preservative formulations, tar color formulations, etc.

In the food composition of the present invention, the MYB, HDAC2, and FOXA2 inhibitors may be included preferably in an amount of 0.00001 to 50 wt% based on the food composition. If the content is less than 0.00001 wt%, the effect is insignificant, and if the content exceeds 50 wt%, an increase in effect compared to the amount used is minimal, which is uneconomical.

In addition, in order to use the MYB, HDAC2, and FOXA2 inhibitors of the present invention in the form of food additives, the MYB, HDAC2, and FOXA2 inhibitors may be prepared and used in the form of tablets, capsules, powders, granules, liquids, pills, etc.

When the composition of the present invention is prepared as beverages, like general beverages, the composition may include various flavoring agents or natural carbohydrates as an additional ingredient. The above-mentioned natural carbohydrates may be used with monosaccharides such as glucose and fructose, disaccharides such as maltose and sucrose, natural sweeteners such as dextrin and cyclodextrin, synthetic sweeteners such as saccharin and aspartame, and the like. A ratio of the natural carbohydrates may be generally about 0.01 to 10 g, preferably about 0.01 to 0.1 g per 100 ml of the composition of the present invention.

In addition, the composition of the present invention may include various nutrients, vitamins, electrolytes, flavoring agents, coloring agents, pectic acid and salts thereof, alginic acid and salts thereof, organic acid, a protective colloidal thickener, a pH adjusting agent, a stabilizer, a preservative, glycerin, alcohols, a carbonic acid agent used in a carbonated drink, or the like. In addition, the composition of the present invention may include pulps for preparing natural fruit juices, fruit juice beverages or vegetable beverages. These ingredients may be used independently or in combination. Although the ratio of these additives is not greatly important, generally, the ratio thereof is selected in a range of 0.01 to 0.1 parts by weight per 100 parts by weight of the composition of the present invention.

As used in the present invention, the "health supplement" or "health functional food" refers to food prepared and processed using raw materials or ingredients with functionality, which are useful for the human body according to the Health Functional Foods Act, and the "functionality" means intake for adjusting nutrients for the structures and functions of the human body or obtaining an useful effect on health applications such as physiological actions.

In another aspect, the present invention relates to a composition for conversion of cancer cells into differentiated normal cells or normal-like cells, including a MYB inhibitor, a HDAC2 inhibitor and a FOXA2 inhibitor and a method for inducing the conversion of cancer cells into differentiated normal cells or normal-like cells, including treating cancer cells with the MYB, HDAC2, and FOXA2 inhibitors *in vitro.*

In yet another aspect, the present invention provides a screening method of a colon cancer therapeutic agent including the following steps:
(a) treating colon cancer cells with a candidate substance;
(b) measuring the expression levels of MYB, HDAC2 and FOXA2 in the colon cancer cells treated with the candidate substance; and
(c) determining the candidate substance as a colon cancer therapeutic agent if the expression levels of MYB, HDAC2 and FOXA2 are lower than that of a control group untreated with the candidate substance.

According to the screening method of the present invention, a candidate substance to be analyzed may first be in contact with cancer cells including the gene or protein. The candidate substance refers to an unknown substance used in screening to test whether the substance affects the expression level of the gene, the amount of protein, or the activity of the protein.

The candidate substance may include chemicals, antisense oligonucleotides, antisense-RNA, siRNA, shRNA, miRNA, antibodies specific for the protein, or natural product extracts, but is not limited thereto.

In the present invention, the candidate substance may include siRNA, shRNA, miRNA, or antibodies specific for MYB, HDAC2 or FOXA2.

Thereafter, the expression level of the gene, the amount of the protein, or the activity of the protein may be measured in the cells treated with the candidate substance. As the measured result, when it is measured that the expression level of the gene, the amount of the protein, or the activity of the protein is decreased, it may be determined that the candidate substance may be used as an agent capable of treating or preventing cancer.

The method of measuring the expression level of the gene or the amount of protein may be performed including a known process of isolating mRNA or protein from a biological sample using a known technique. The "biological sample" refers to a sample collected from a living body of which the gene expression level or protein level is different from that of the control group according to the degree of cancer development and progression. Examples of the sample may include tissue, cells, blood, serum, plasma, saliva, and urine, but are not limited thereto.

The expression level of the gene is preferably measured by measuring the level of mRNA, and methods for measuring the mRNA level include reverse transcription polymerase chain reaction (RT-PCR), real-time reverse transcription polymerase chain reaction, RNase protection assay, Northern blot, DNA chips, etc., but are not limited thereto.

The protein level may be measured using an antibody. In this case, the marker protein in the biological sample and a specific antibody thereto form a combination, that is, an antigen-antibody complex, and the formed amount of the antigen-antibody complex may be measured quantitatively through the signal size of a detection label. The detection label may be selected from the group consisting of enzymes, fluorophores, ligands, luminescent substances, microparticles, redox molecules and radioisotopes, but is not limited thereto. Analysis methods for measuring the protein levels include Western blot, ELISA, radioimmunoassay, radioimmunodiffusion, Ouchterlony immunodiffusion, rocket immunoelectrophoresis, tissue immunostaining, immunoprecipitation assay, complement fixation assay, FACS, protein chips, etc., but are not limited thereto.

In another aspect, the present invention provides a method for treating colon cancer including administering a MYB inhibitor, a HDAC2 inhibitor and a FOXA2 inhibitor to a subject.

As used in the present invention, the term "subject" refers to an individual having a cancer disease, preferably mammals such as horse, sheep, pig, goat, dog, including humans having a cancer disease, and preferably humans.

Since the method of the present invention uses the MYB, HDAC2, and FOXA2 inhibitors described above, the description of duplicated contents will be omitted to avoid excessive complexity of the present specification.

### [Modes of the Invention]

Hereinafter, the present invention will be described in more detail with reference to Examples. These Examples are only illustrative for the present invention, and it will not be understood that the scope of the present invention is limited by these Examples.

### Example 1: Construction of network of key genes in cell differentiation process

Cells change from a high stemness state to a differentiated state, and gene expression changes during this process. By analyzing single-cell transcriptome data that change during the cell differentiation process through pseudo time analysis, a method capable of inferring structural information of a gene network and inferring interaction logic of genes involved in the differentiation process was derived.

A logic network was constructed using a cell group corresponding to a pseudo time in which cells differentiated into enterocytes, and key regulators in the cell differentiation process were discovered through simulation.

### Pseudo-time and Pseudo-state

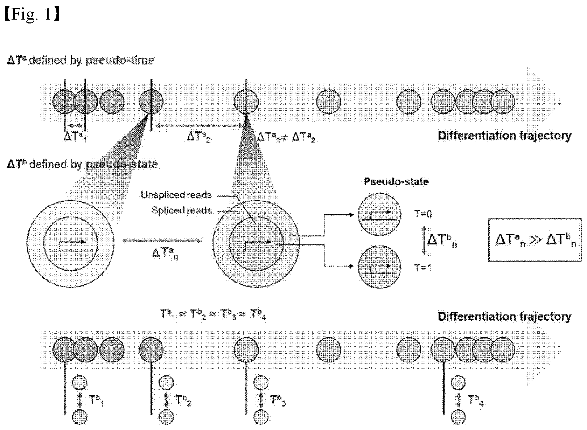

FIG 1 is a schematic diagram illustrating a process of lining up single cells in a differentiation process according to a pseudo time and dividing a state in which genes are changed by genetic regulators within a single cell, rather than in front and behind the single cell where each pair of changes is lined up, into two states based on the characteristics of mRNA that appear in the physically separated nucleus and cytoplasm.

Specifically, the process is subjected to a process of separating a single cell into two states, that is, Pseudo-states, according to a pseudo time corresponding to the process in which the cells change from a high stemness state to a differentiated state (enterocyte).

Each pseudo-state is distinguished in the following method.

When producing single-cell RNA seq data, approximately 15% of reads in each cell are located in the intron region (unspliced reads). It was known that a gene located in this way was derived from immature RNA, which was spatially divided and existed only in the nucleus. This gene was newly generating nascent RNA and was used as a standard to measure the activity level in each transcription factor. This state may be represented as T = 1 and corresponds to a state where the gene is turned on.

A value consisting of exonic RNA in the cytoplasm where the spliced reads are located, consists of actual mature mRNA, which is then translated into protein. This state may be represented as T = 0 and correspond to a state where the gene is turned off.

Based on these two RNAs with different characteristics, each pseudo cell was set and named as a Pseudo-state.

Since immature RNA in the nucleus changes into a mature form and comes out into the cytoplasm to be translated into protein, the mature RNA coming out into the cytoplasm may be equivalent to transcription factors in the same amount. In other words, the amount of mRNA expressed by transcription factors may be estimated. Based on this, it is possible to infer logic that expresses the dynamics of genes based on the process in which nascent RNA, i.e. immature RNA, which begins to be produced inside the nucleus of each cell, is transcribed by transcription factors, construct a gene network by integrating the logic at a system level, and find a candidate regulator of a target gene by analyzing the network.

### Inference of latent regulator for each gene

A method for deriving transcription factor-gene pairs was performed. The process of inferring the genetic regulator of each gene includes a process of finding a candidate regulator of a gene by calculating conditional mutual information for a pair of one gene (source) and another gene (target) while comparing the two gene states defined above, i.e., pseudo-states, in the pseudo-time order.

In the process, the present inventors applied the concept of moving windows. In this concept, to comprehensively analyze a gene network that occurs early and a gene network that occurs late when aligned according to a change in a single cell, a gene network was constructed by repeating sections grouping 400 single cells listed according to pseudo time.

The sections become the number (n) obtained by dividing the total number of single cell sources used by 400. Each section shares some cells with the previous and subsequent sections. In this process, n networks constructed for each section are merged and used as a single network. The network constructed in this way includes not only a regulatory relationship that appears consistently throughout the entire network, but also a regulatory relationship that appears characteristically in each section. That is, when the gene determines the state of any cell, both the early and late regulatory relationships of the gene are important, and when CMI is analyzed entirely without dividing the sections, it is possible to solve a problem in which a feedback correlation of the gene regulatory relationship is not properly reflected.

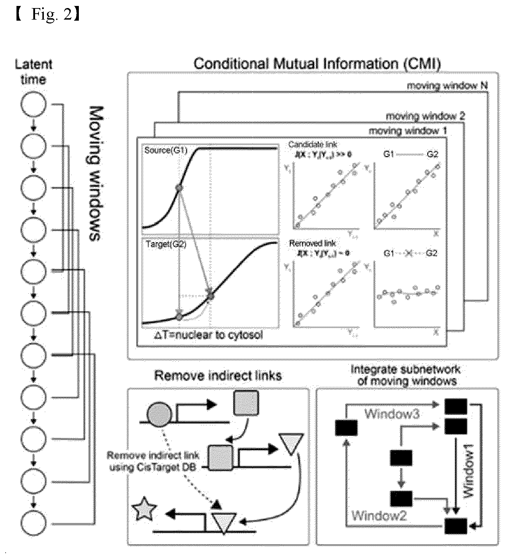

FIG. 2 is a diagram illustrating a process of deriving candidate regulators through conditional mutual information (CMI) analysis of the effect of a source gene (G1) on the mature RNA expression level of an actual target gene (G2), i.e., a change from nuclear to cytosol (ΔT = nuclear to cytosol), in each section constructed by section.

As shown in Fig. 2, when cells were fixed in one state, through a relationship in which when the source gene (G1) increased, the target gene (G2) increased, it may be considered that the source gene (G1) regulates the target gene (G2). In this process, to eliminate cases where the source gene (G1) does not directly regulate but indirectly regulates the target gene (G2), a RcisTarget database, a public database, was used. In the RcisTarget database, genes that may not be directly bound may be filtered and removed based on binding motif information of the transcription factors and a DNA base sequence of the gene among their connections. Through this process, gene candidate regulator data were constructed by integrating subnetworks of the moving windows to perform mutual feedback.

Thereafter, a network model is constructed using a Boolean function from the derived gene candidate regulator data.

### Binary conversion of gene expression levels

Since RNA sequencing data are continuous values, these values need to be binarized to prepare a truth table.

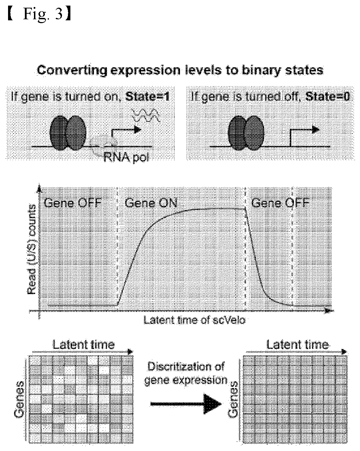

FIG. 3 illustrates a process of placing a gene activity level in two states, i.e., on (State = 1) and off (State = 0), locating two types of binary codes in different groups, and binarizing each group.

The expression of the gene is determined as turned on, and no expression of the gene is determined as turned off, but the gene activity level is determined by determining that the gene is expressed only up to a point where a graph of read (U/S) number on a graph (middle image) according to latent time forms an increasing curve (Gene on, turn on), and that the gene is not expressed in the remaining points (Gene off, turn off). As described in FIG. 1 above, when unspliced RNA exists, unspliced RNA is bound with a transcription factor and RNA transcription occurs, which may be classified as a turn on (T = 1) in which the gene is expressed. On the other hand, when RNA exists, but unspliced RNA does not exist, the gene may be classified as turned off (T = 0) because the previously transcribed RNA remains even though the gene has been turned off.

Through this process, all genes containing transcription factors (TFs) are binarized into 1 or 0 by reflecting the on/off state of the gene. The bottom of FIG. 3 illustrates that RNA sequencing data in all single-cell genes are converted to 1 or 0 according to latent time.

### Identification of Boolean function and reconstruction of Boolean network model

It may be assumed that mature RNA present in the cytoplasm is similar to the amount of regulators through a transcription-translation process. Accordingly, in a pseudo-state, the mature RNA may be considered as the amount of regulators, i.e. transcription factors (TF). Based on this, the amount of mRNA that binds to TFs to induce expression may be estimated, and it is assumed that the nascent RNA (immature RNA) that begins to be produced inside the nucleus of each cell is a gene regulated by the previously inferred regulators (TF).

Then, by integrating all the Input (Regulator) - Output (nascent RNA) pairs based on these changed values, a simulatable Boolean function is made using a Quine-McCluskey algorithm.

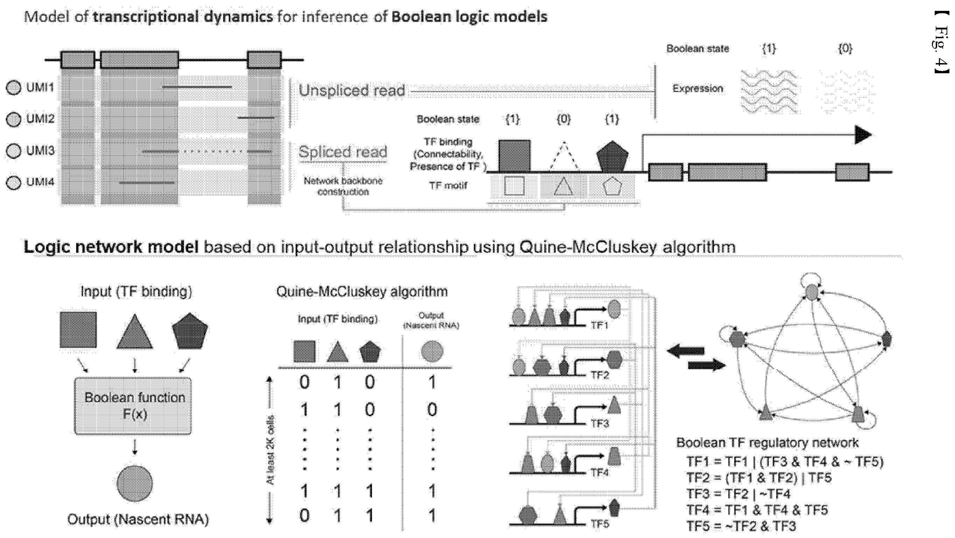

FIG. 4 illustrates a model of transcriptional dynamics for inference of Boolean logic models and a logic network model based on an input-output relationship using a Quine-McCluskey algorithm.

In the pseudo-state, mature RNA (exonic RNA), TF corresponding to T = 0 is input onto the truth table, and immature RNA (intronic RNA) corresponding to T = 1 is output.

When preparing the truth table, rows with the same input but different outputs may occur frequently, but rows with a small number thereof may be considered noise. Thus, the truth table was completed by selecting a plurality of input-output rows, and a final Boolean function was made by making a Boolean expression of minimal form through a QM algorithm, which is an algorithm for reducing Boolean expressions.

Referring to FIG. 4, when assuming a TF motif, which may exist as a transcription factor (TF) in a region where the spliced reads existing in the cytoplasm are transcribed into a protein, as one machinery capable of turning on/off a gene, in the machinery, if the gene is turned on and RNA is transcribed, there will be unspliced reads, and if the gene has been turned on, but previously transcribed RNA remains, there may be no unspliced reads. Therefore, the Boolean state is 1 if unspliced reads exist, and 0 if the unspliced reads do not exist.

This binary conversion is performed for each transcription factor (TF) involved in the moment when RNA is expressed or not, and a logic form may be constructed as 1 when the gene is expressed and 0 when the gene is not expressed, according to a pattern of TFs expressed as 1 or 0. As a result, when a certain type of TF exists, a pattern of whether the gene is expressed (1) or not expressed (0) is derived. The presence of gene expression may be identified according to a pattern of each transcription factor with 99% accuracy when performed on approximately 2,000 or more pairs.

For example, TF1 may be expressed (1) through regulatory pairs such as TF1; or TF3 and TF4 binding and TF5 non-binding. When describing the Boolean expression, when TF2 and TF3 need to be 1 at the same time (and) and TF5 needs to be 0, TF1 is expressed.

When the process is repeated for all genes in each pseudo-state to be applied to all pairs and the desired state of the cell is fixed in this process and applied with a BNsimpleReduction algorithm, it is possible to generate regulatory relationship logic of source genes and target genes closely involved in the desired state of the cells.

The constructed gene network is then used in a mathematical regulatory relationship inference step to find a transcription factor (TF) for each gene.

In this process, a parallel computing method was applied to optimize computational efficiency. Parallel computing is useful when processing large data sets or performing complex calculations, and means parallelizing multiple calculations by assigning each task to each core of a central processing unit of the computer. By applying this method, the calculations were assigned and parallelized to calculate the control relationship logic for each CPU and the logic inference time was significantly reduced.

### Example 2: Discovery of MYB/HDAC2/FOXA2 differentiation regulation targets in colon cells

The networks of key genes constructed in Example 1 were simulated using single-cell transcriptome data of colon cells samples.

Specifically, in samples collected from six patients diagnosed with colon tumors (Y Wang, 2019, Cited by 174, J Exp Med (2020) 217 (2): e20191130), a total of 4252 single-cell data from the enterocyte portion corresponding to absorptive pathway cells were used as a source among single-cells obtained from two colon samples and two rectum samples.

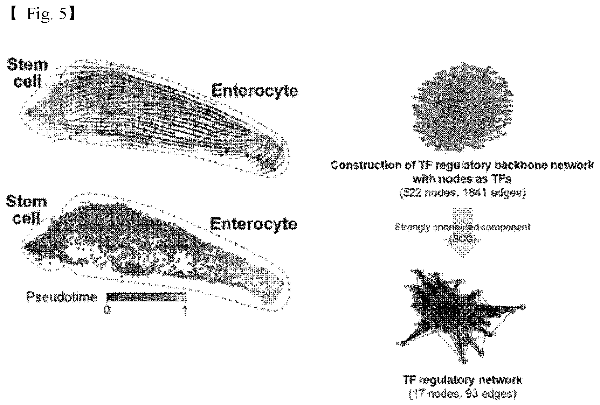

Referring to FIG. 5, as a result of performing conditional mutual information (CMI) analysis on a total of 20 sections (windows) obtained by grouping 400 single cells sorted according to a pseudo time of changing from a high stemness cell (Stem cell) to a differentiated cell (Enterocyte), a network of key genes consisting of 522 genes was constructed. At this time, a TF gene network consisting of 17 genes was reconstructed using Strongly connected component (SCC) to isolate genes with indirect links that were not filtered out.

The 17-gene data were binarized according to the method of FIG. 3, and a truth table was prepared using mature RNA of each state as input, and then the final Boolean function in the minimum form was generated through a QM algorithm. Thereafter, by applying the BNsimpleReduction algorithm developed by the present inventors, a key gene regulatory network with logic consisting of 13 genes with a strong regulatory relationship was constructed (FIG. 6).

The constructed gene network model dynamics were verified by comparing and analyzing the dynamics of the single-cell data used (FIG. 7).

First, the binarized single-cell data of each gene included in the network were indicated according to pseudo-time to confirm the ON and OFF state changes of each gene. In addition, to define the binary state corresponding to each cell type, the average activity of each gene of stem cells and late enterocytes (the last 300 cells in pseudo-time) classified at the single-cell processing step was calculated, and the calculated average activity was compared with the average activity of each gene of all cells to determine the binarization state corresponding to each cell type (FIG. 7A).

Thereafter, dynamics of convergence from a single initial state to a stable state (attractor) of the network model were compared. As a result of mapping a changing state to correspond to the closest cell state by performing a simulation from the initial state corresponding to the stem cell, it was confirmed that the dynamics of convergence from the initial state to the stable state were associated with original single-cell data, and thus it was confirmed that the dynamics of the original data were well reflected to transient dynamics of the convergence process of the gene network model. Referring to FIG. 7B, it may be seen that the simulation process is consistent with the pseudo-time direction.

In addition, as a result of identifying all the point attractors of the gene network model and mapping the point attractors to correspond to the closest cell state to each point attractor, referring to FIG. 7C, the point attractors are widely distributed across the entire data, and the relative basins are also relatively evenly distributed. In addition, a distance from the closest cell state is mostly 0 or 1, which means that only one gene has a difference, and the network state is very similar to the actual cell state, thereby validating the mapping.

In addition, by calculating and mapping the similarity between the state of each cell type determined above and the point attractors, it was confirmed that the similarity of stem cells decreased and the similarity of enterocytes increased according to the pseudo-time change (FIG. 7D).

These results support that the stable state dynamics of the gene network model well reflect the dynamics of actual single-cell data.

These results have verified that the constructed gene network model may simulate combinations of key gene regulators to be converted to a desired state.

BNsimpleReduction is an efficient global stabilization algorithm for discovering target controls by applying FVS known to be controlled to all attractor states after reducing a network by a method in which in a Boolean network, only nodes and links that play the most important role in preserving the dynamic information for a single desired attractor state are left and the rest are removed.

When the number of targets found by the BNsimpleReduction algorithm is large, all combinations are perturbed, respectively, and then an attractor simulation is performed to compare the obtained average activity with the desired state, and combinations with a high similarity score but a small number are discovered as final targets.

In the gene network consisting of 13 genes derived above, a total of five targets were derived for global stabilization using the BNsimpleReduction algorithm.

Referring to FIG. 8A, the network consisting of the 13 genes is reduced and a minimum feedback set is identified. Specifically, as each simulation result of exploring a total of five targets and applying all possible combinations of the targets, when the similarity between the stable state of the network and the desired state (enterocyte point attractor) was calculated, a similarity score of approximately 95% or more was achieved by turning off the three optimal targets MYB, HDAC2, and FOXA2.

As a result of identifying the phenotype landscape of the network while increasing the number of optimal three-target combinations, as shown in FIG. 8B, it was confirmed that convergence to the desired state (enterocyte point attractor) did not occur with one or two combinations, and only the simultaneous application of the three targets MYB, HDAC2, and FOXA2 converged the undifferentiated network point attractors to the desired state (enterocyte point attractor).

Through the above process, the MYB/HDAC2/FOXA2 differentiation regulation target was discovered, and the function of the MYB/HDAC2/FOXA2 differentiation regulation target was verified through experiments to be described below.

### Example 3: Reversion of cancer cells according to MYB/HDAC2/FOXA2 control

### 3-1. Cancer cell proliferation inhibitory effect

It was observed whether the proliferation ability of cancer cell lines was reduced under the control of the cell differentiation regulators MYB, HDAC2 or FOXA2 selected in Example 2.

First, colon cancer cell lines HCT 116, HT 29, and CACO 2 were transfected by an Invitrogen RNAi MAX Forward Transfection method.

Specifically, each cell line was seeded in 24 wells in the number of 1 × 10⁵, and the next day, a transfection mixture was prepared and added to each well. The transfection mixture was prepared as follows. Each siRNA was added to 50 µl of Opti-MEM to obtain a final concentration of 10 nM and mixed. 1 µl of RNAi MAX was added and mixed to 50 µl of Opti-MEM, and then mixed with a diluted siRNA and RNAi max and incubated at room temperature for about 10 minutes. When 100 µL of the mixture was dropped into each well, the final volume became 600 µL and the final concentration became 10 nM.

siRNAs used to inhibit MYB, HDAC2, and FOXA2 were shown in Table 1.

**[Table 1]**

| siRNA | Sense sequence | Anti-sense sequence |
|---|---|---|
| si-MYB | GGUCGAACAGGAAGGUUAU (SEQ ID NO: 1) | AUAACCUUCCUGUUCGACC (SEQ ID NO: 2) |
| si-HDAC2 | GAAGAUCCAGACAAGAGAAU UUCUA (SEQ ID NO: 3) | UAGAAAUUCUCUUGUCUGGA UCUUC (SEQ ID NO: 4) |
| si-FOXA2 | UGAACGGCAUGAACACGUA (SEQ ID NO: 5) | UACGUGUUCAUGCCGUUCA (SEQ ID NO: 6) |

The transfected MYB, HDAC2 or FOXA2 knockdown cell line was transferred to a 96-well plate after 24 hours and placed in a real-time cell line observation device (InCucyte; inCu saFe, Panasonic), and photographed at 3-hour intervals, and the cell proliferation was measured and shown in FIG. 7.

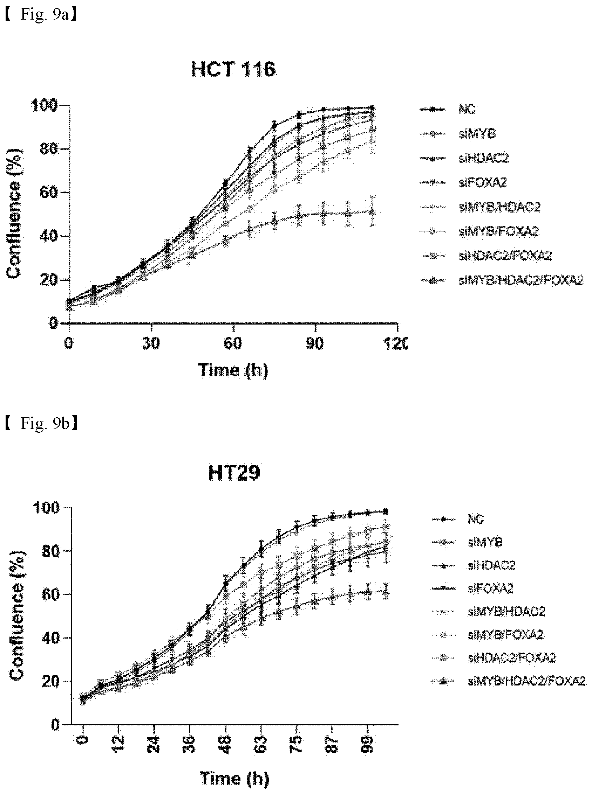

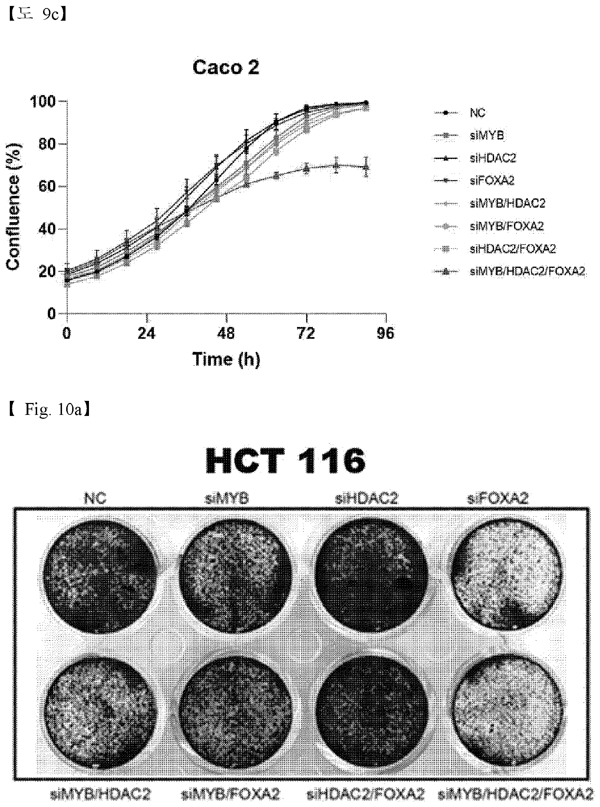

Referring to FIGS. 9A to 9C, (a) HCT 116 cell line, (b) HT 29 cell line, and (c) CACO 2 cell line all exhibited a synergic effect of significantly reducing the cell proliferation when MYB, HDAC2 and FOXA2 were simultaneously inhibited (siMYB/HDAC2/FOXA2).

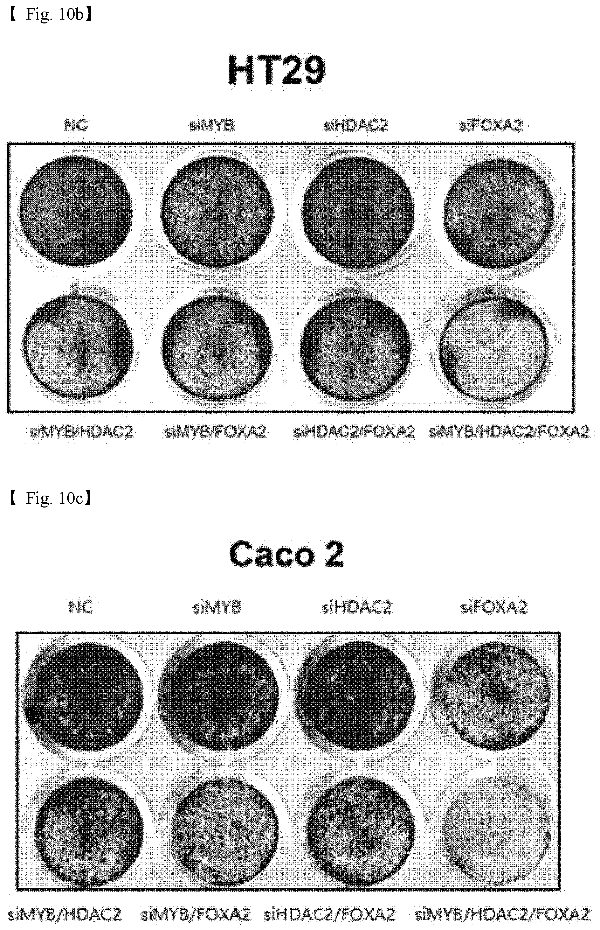

In addition, 5 days after transfection, images of the MYB, HDAC2, or FOXA2 knockdown cell line stained with crystal violet and taken are shown in FIG. 10.

Referring to FIGS. 10A to 10C, (a) HCT 116 cell line, (b) HT 29 cell line, and (c) CACO 2 cell line all exhibited a proliferation inhibition synergic effect of significantly reducing the cell number when MYB, HDAC2 and FOXA2 were simultaneously inhibited (siMYB/HDAC2/FOXA2). In particular, the synergic effect was excellent in CACO 2 cell line.

### Example 4: Reversion of cancer cells according to MYB/HDAC2/FOXA2 control

It was confirmed reversion of cancer cell lines according to the control of the cell differentiation regulator MYB, HDAC2 or FOXA2 selected in Example 2.

### 4-2. Effect of increasing differentiation ability of cancer cells

The mRNA expression levels of enterocyte differentiation markers were determined in the MYB, HDAC2, or FOXA2 knockdown cell line. The sequences of primers used in qRT-PCR to confirm the expression of each marker were shown in Table 2.

**[Table 2]**

| Gene symbol | Sense sequence | Anti-sense sequence |
|---|---|---|
| GAPDH | 5'- TGATGACATCAAGAAGG TGGTGAAG-3' (SEQ ID NO: 7) | 5- TCCTTGGAGGCCATTGGGCCAT -3’ (SEQ ID NO: 8) |
| MYB | 5'- GGGAACAGATGGGCAG AAATCG 3'(SEQ ID NO: 9) | 5’- GCTGGCTTTTGAAGACTCCTGC- 3’ (SEQ ID NO: 10) |
| HDAC2 | 5’- GAGGTGGCTACACAATC CSTA-3’(SEQ ID NO: 11) | 5- ACACCAGGTGCATGAGGTAAC- 3’ |
| | | (SEQ ID NO: 12) |
| FOXA2 | S’- GGAACACCACTACGCCT TCAAC-3’(SEQ ID NO: 13) | 5- AGTGCATCACCTGTTCGTAGGC -3' (SEQ ID NO: 14) |
| AQP7 | 5’- TTAACCACTTTGCAGGG GA-3’(SEQ ID NO: 15) | 5-TTCTTGTCGGTGATGGCGAA- 3' (SEQ ID NO: 16) |
| NBC1 | 5’- GGGGAAAGCCAAGTCCT ACC-3’(SEQ ID NO: 17) | 5-CCAGCAATCAGGTCGTGTCT 3' (SEQ ID NO: 18) |
| AE2 | 5’- AACCTTCAGGAGCGAAG ACG-3’(SEQ ID NO: 19) | 5-TCTTCCTCACCAAGTGTCGC- 3' (SEQ ID NO: 20) |
| NHE3' | 5’- GCCTATGCCTTGGTGGT ACT-3’(SEQ ID NO: 21) | 5-CCGGCCATGTAGCTTGTCAT- 3' (SEQ ID NO: 22) |
| VILLIN | 5’ CTGGACCTTGGGAAGCT TATT-3’(SEQ ID NO: 23) | 5'- AAAGAGATCCGAGACCAGGA- 3' (SEQ ID NO: 24) |
| CREB3' | 5'- GAAGCCTCTTGACCATA GACC-3'(SEQ ID NO: 25) | 5'- GGAGGTCTTTCACGGTGAGATT G-3' (SEQ ID NO: 26) |
| FABP2 | S’- CAATCTAGCAGACGGAA CTGAAC-3’(SEQ ID NO: 27) | 5’- CGGACAGTATTCAGTTCGTTTC C-3’ (SEQ ID NO: 28) |
| MAF | 5- AGAAGTTGGTGAGCAGC GGCTT-3'(SEQ ID NO: 29) | 5- CACTGATGGCTCCAACTTGCGA -3’ (SEQ ID NO: 30) |
| CSLC26A3' | 5'- GGCAGATTTGGCGAAAG GACA-3'(SEQ ID NO: 31) | 5- GGTCCTGAACACGATGGTTAGC -3' (SEQ ID NO: 32) |
| NRIH4 | 5’- ACTTCCGTCTGGGCATT CTGAC-3’ (SEQ ID NO: 33) | 5- GCTGTAAGCAGAGCATACTCCT C-3’ (SEQ ID NO: 34) |

Specifically, 48 hours after transfection of the HCT 116, HT 29, and CACO 2 cell lines in Example 3, RNA was extracted using a Total RNA Extraction kit (iNtRON). cDNA was synthesized by reverse transcription using 1 µg of RNA extracted with the Solgent RTase kit.

In the cDNA synthesis method, distilled water was added to 1 µg of RNA to make a total volume of 10 µL. 1 µL of a primer mixture containing a 50 µM oligo dT primer and a 50 Um random hexamer primer was added and incubated at 65°C for 5 minutes. After 5 minutes, the mixture was added in ice and cooled for 2 minutes or more. 4 µL of a 5xRT reaction buffer (containing 10 mM dNTP), 1 µL of 8 mM DTT, and 1 µL of DiaStar-TM RTase were added, and then added with distilled water to make a total volume of 9 µL. The RTase mixture prepared above was placed in an ice-cooled tube and then reverse transcription was performed at 42°C for 1 hour.

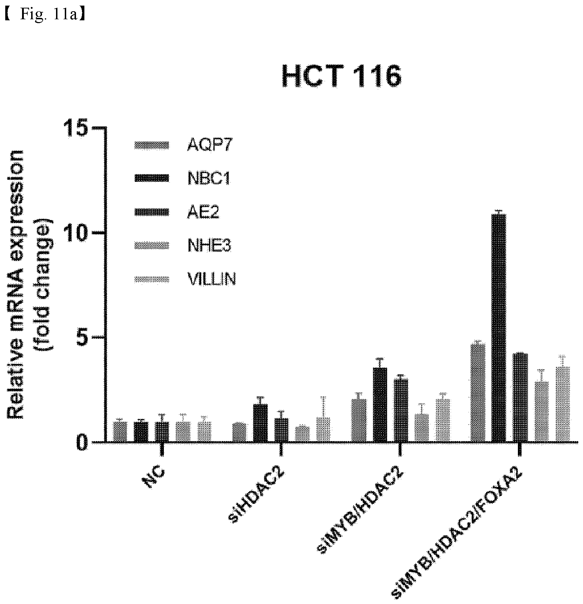

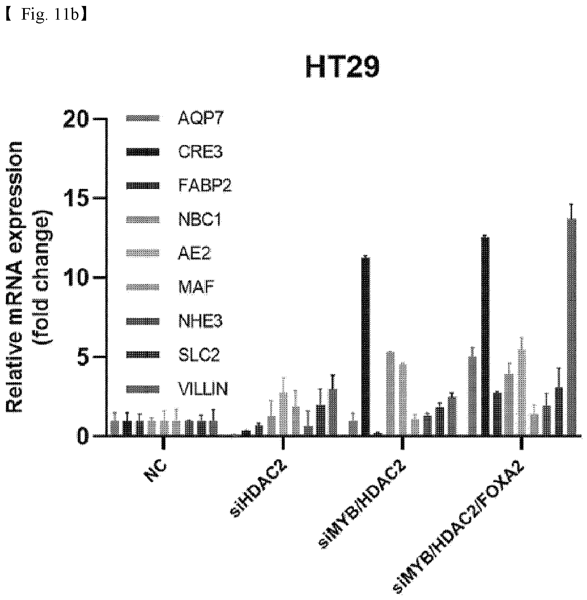

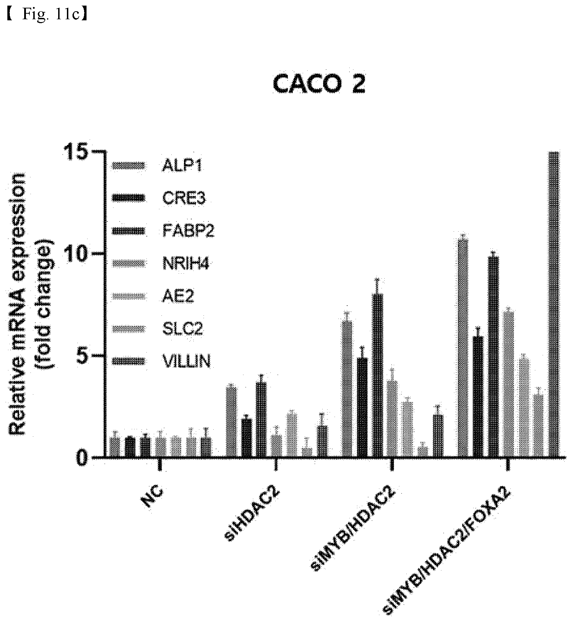

To confirm the expression of enterocyte differentiation markers in Table 2, qRT-PCR was performed for synthesized cDNA. The synthesized cDNA was diluted to 1/10, and then 1 µL of the diluted cDNA was placed into a 96-well dedicated for qRT-PCR, added with 2xCYBER GREEN and 0.2 µM of the primer, and added with distilled water to make a total volume of 20 µL, and then performed at an annealing temperature of 65°C, and a graph drawn using delta Ct values is shown in FIG. 11.

Referring to FIGS. 11A to 11C, (a) HCT 116 cell line, (b) HT 29 cell line, and (c) CACO 2 cell line all exhibited a synergic effect of increasing the expression of enterocyte differentiation markers when MYB, HDAC2 and FOXA2 were simultaneously inhibited (siMYB/HDAC2/FOXA2).

As these results, it was confirmed that cancer cells were reverted by differentiating the colon cancer cells into normal cells by simultaneous inhibition of the differentiation regulatory target combination MYB/HDAC2/FOXA2.

### Example 5: In vivo cancer inhibition effect according to MYB/HDAC2/FOXA2 control

Animal experiments were conducted using methods approved by the Animal Ethics Committee of the Korea Advanced Institute of Science and Technology, and tumor formation according to MYB, HDAC2, and FOXA2 inhibition was observed using an animal model transplanted with colon cancer cells.

siRNAs used to inhibit MYB, HDAC2, and FOXA2 were shown in Table 3.

**[Table 3]**

| shRNA | Sequence |
|---|---|
| sh-MYB | CCGGGCTATCAAGAACCACTGGAATCTCGAGATTCCAGTGGTT CTTGATAGCTTTTTG (SEQ ID NO: 35) |
| sh-HDAC2 | CCGGCAGTCTCACCAATTTCAGAAACTCGAGTTTCTGAAATTG GTGAGACTGTTTT (SEQ ID NO: 36) |
| sh-FOXA2 | CCGGGGAACACCACTACGCCTTCAACTCGAGTTGAAGGCGTA GTGGTGTTCCTTTTTG (SEQ ID NO: 37) |

Specifically, the shRNA in Table 3 was transduced into colon cancer cell lines HCT 116 and CACO 2 to simultaneously control MYB, HDAC2, and FOXA2 and inhibit the expression.

The two types of cell lines with inhibited expression were transplanted into immuno-deficient nude mice, in which a colon cancer cell line (5 × 10⁶) with normal expression of the MYB/HDAC2/FOXA2 combination was transplanted into the left back, and a colon cancer cell line (5 × 10⁶) with inhibited expression of the MYB/HDAC2/FOXA2 combination was transplanted into the right back.

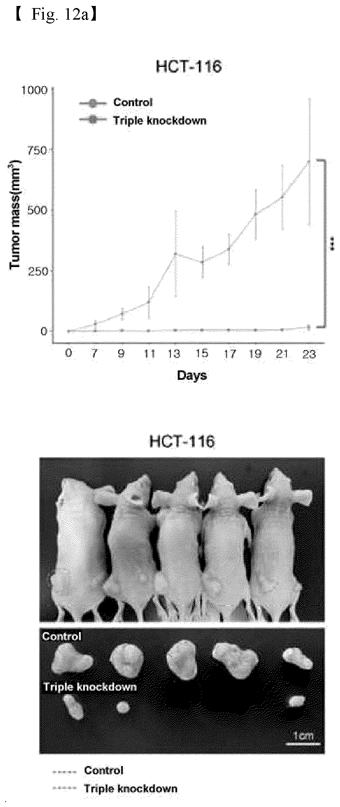

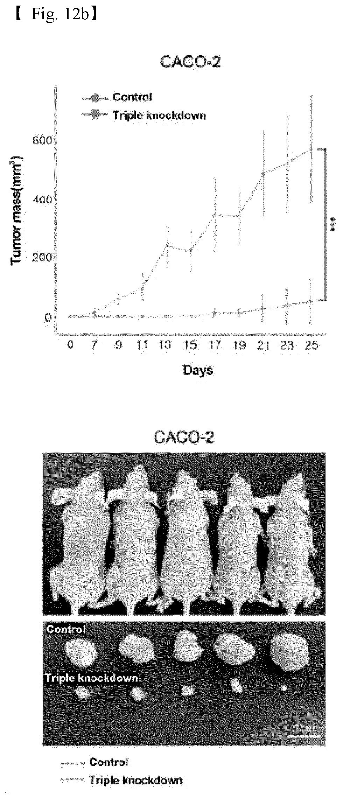

The size of the tumors formed was measured every two days from 7 days after transplantation. The size was set by defining the longest point as a long axis and the shortest point as a short axis, and the volume of each cancer cell (0.5 × long axis × short axis) was measured. The results measured for 23 to 25 days in this way were shown in FIG. 12.

In both mice transplanted with the HCT 116 cell line in FIG. 12A and the CACO 2 cell line in FIG. 12B, it was confirmed that the tumor volume was significantly inhibited in a triple knockdown experimental group transplanted with a cell line in which the expression of the MYB/HDAC2/FOXA2 combination was inhibited, compared to a control group transplanted with a cell line in which the MYB/HDAC2/FOXA2 combination was normally expressed (*** : p < 0.001, Wilcoxon rank sum test).

In particular, referring to FIG. 12A, it may be confirmed that no tumors were formed in the third and fourth mice.

As these results, it was confirmed that the inhibition of the differentiation regulation target MYB/HDAC2/FOXA2 combination discovered through the network of key genes constructed by the present inventors exhibited a synergy to significantly inhibit tumor formation or growth due to a characteristic of reverting the differentiation of cancer cells into normal cells.

## Claims

1. A method for constructing a network of key genes comprising:
a) sorting single cells according to a pseudo time in which cells with high stemness differentiate into differentiated cells;
b) separating each single cell sorted in step a) into a turn on Pseudo-state where each gene is expressed and a turn off Pseudo-state where each gene is not expressed, according to characteristics of immature RNA (nascent RNA) in the nucleus and mature RNA (exonic RNA) in the cytoplasm using single-cell transcriptome data;
c) deriving gene pairs that affect mutual expression by dividing the single cells sorted according to the pseudo time in step a) into sections so that X cells are included per group over time and performing conditional mutual information (CMI) analysis between genes over time using a moving windows analysis method, wherein X = 100 to 500;
d) constructing candidate regulator data of target genes with mutual feedback by excluding gene pairs that are not direct gene regulatory pairs from among the gene pairs derived through step c) using a CisTarget DB;
e) generating a simulatable control relationship logic by applying a truth table prepared by discretizing the candidate regulator data of the target genes in step d) to all gene pairs in each pseudo-state; and
f) selecting a final target that achieves global stabilization by using a BNsimpleReduction algorithm in the generated regulation relationship logic of step e).

2. The method of claim 1, wherein the mature RNA (exonic RNA) of step b) is considered as the amount of transcription factor, which is a regulator, and the immature RNA (nascent RNA) is considered as a gene regulated by the regulator.

3. The method of claim 1, wherein the CMI analysis of step c) is performed by dividing sections to include 400 cells and repeating moving window analysis.

4. The method of claim 1, wherein when the binarization of step d) corresponds to a section that forms a curve where the number of unspliced reads/spliced reads increases with latent time, the section is determined as turned on, State = 1, where the gene is expressed and the remaining sections are determined as turned off, State = 0, where the gene is not expressed.

5. The method of claim 1, wherein in the truth table of step e), the mature RNA (exonic RNA) is input and the immature RNA (nascent RNA) is output.

6. The method of claim 1, wherein the final target for achieving the global stabilization of step f) is a combination with a similarity score of 90% to 100% by comparing the similarity scores between the stable state and the desired state of the network and a small number of regulators.

7. A pharmaceutical composition for the prevention or treatment of colon cancer, comprising a MYB inhibitor, a histone deacetylase 2 (HDAC2) inhibitor, and a forkhead box protein A2 (FOXA2) inhibitor.

8. The pharmaceutical composition for the prevention or treatment of colon cancer of claim 7, wherein the MYB, HDAC2 and FOXA2 are a combination of key regulators that induce synergy in the differentiation of cancer cells.

9. The pharmaceutical composition for the prevention or treatment of colon cancer of claim 7, wherein the inhibitor is at least one selected from the group consisting of antisense oligonucleotide, small interference RNA (siRNA), short hairpin RNA (shRNA), microRNA (miRNA), and ribozyme, that bind complementarily to mRNA of a MYB, HDAC2 or FOXA2 gene.

10. The pharmaceutical composition for the prevention or treatment of colon cancer of claim 7, wherein the inhibitor is at least one selected from the group consisting of a compound, a peptide, a peptide mimetic, a substrate analog, an aptamer, and an antibody, that specifically bind to a MYB, HDAC2 or FOXA2 protein.

11. The pharmaceutical composition for the prevention or treatment of colon cancer of claim 7, wherein the composition induces reversion of cancer cells into differentiated normal cells or normal-like cells.

12. An anticancer adjuvant comprising a MYB inhibitor, a histone deacetylase 2 (HDAC2) inhibitor, and a forkhead box protein A2 (FOXA2) inhibitor.

13. A food composition for the prevention or alleviation of colon cancer, comprising a MYB inhibitor, a histone deacetylase 2 (HDAC2) inhibitor, and a forkhead box protein A2 (FOXA2) inhibitor.

14. A method for inducing conversion of colon cancer cells into differentiated normal cells or normal-like cells, comprising treating colon cancer cells with a MYB inhibitor, a histone deacetylase 2 (HDAC2) inhibitor, and a forkhead box protein A2 (FOXA2) inhibitor *in vitro.*

15. A screening method of a colon cancer therapeutic agent comprising the following steps:
(a) treating colon cancer cells with a candidate substance;
(b) measuring the expression levels of MYB, HDAC2 and FOXA2 in the colon cancer cells treated with the candidate substance; and
(c) determining the candidate substance as an agent for treating colon cancer if the expression levels of MYB, HDAC2 and FOXA2 are lower than that of a control group untreated with the candidate substance.

16. A method for treating colon cancer, comprising administering to a subject a MYB inhibitor, a histone deacetylase 2 (HDAC2) inhibitor, and a forkhead box protein A2 (FOXA2) inhibitor.
